(19) Europäisches Patentamt
European Patent Office
Office européen des brevets

(11) **EP 4 748 383 A1**

## (12) EUROPEAN PATENT APPLICATION
published in accordance with Art. 153(4) EPC

(43) Date of publication:
**27.05.2026 Bulletin 2026/22**

(21) Application number: **24862867.9**

(22) Date of filing: **05.09.2024**

(51) International Patent Classification (IPC):
**A61K 31/726** (2006.01)     **A61K 9/08** (2006.01)
**A61K 47/18** (2017.01)     **A61K 47/20** (2006.01)
**A61K 47/22** (2006.01)     **A61P 27/02** (2006.01)

(52) Cooperative Patent Classification (CPC):
**A61K 9/08; A61K 31/726; A61K 47/18;**
**A61K 47/20; A61K 47/22; A61P 27/02**

(86) International application number:
**PCT/JP2024/031846**

(87) International publication number:
**WO 2025/053216 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.09.2023 JP 2023144198**

(71) Applicant: **Rohto Pharmaceutical Co., Ltd.
Osaka-shi, Osaka 544-8666 (JP)**

(72) Inventor: **NAKATA Atsuko
Osaka-shi, Osaka 544-8666 (JP)**

(74) Representative: **Mewburn Ellis LLP
Aurora Building
Counterslip
Bristol BS1 6BX (GB)**

## (54) OPHTHALMIC COMPOSITION

(57)     The present invention relates to an ophthalmic composition containing (A) at least one selected from the group consisting of chondroitin sulfate and a salt thereof, having a weight-average-molecular weight of 1,000 to 20,000, and (B) at least one selected from the group consisting of a water-soluble vitamin, an amino acid, and a salt thereof.

EP 4 748 383 A1

## Description

### Technical Field

The present invention relates to an ophthalmic composition.

### Background Art

Chondroitin sulfate or a salt thereof is a type of acidic mucopolysaccharide and is known to be used for preventing corneal dryness due to its physiological viscosity. (For example, Non Patent Literature 1).

### Citation List

### Non Patent Literature

Non Patent Literature 1: Package insert for ophthalmic chondroitin sulfate preparation, Chondron (registered trademark) eye drop 1%, Chondron (registered trademark) eye drop 3% (revised in November 2007)

### Summary of Invention

### Technical Problem

An object of the present invention is to provide a novel ophthalmic composition containing sodium chondroitin sulfate having a specific weight-average-molecular weight (1,000 to 20,000).

### Solution to Problem

The present inventors have found that an ophthalmic composition formulated with sodium chondroitin sulfate having a weight-average-molecular weight of 1,000 to 20,000 and a water-soluble vitamin or an amino acid can surprisingly reduce the elastic modulus of soft contact lenses. In particular, ophthalmic compositions that are OTC drugs are generally usable even when wearing contact lenses. Those that have benefits for contact lenses or the wearing thereof are useful as ophthalmic compositions.
The present invention provides, for example, the following inventions.

[1] An ophthalmic composition containing (A) at least one selected from the group consisting of chondroitin sulfate and a salt thereof, having a weight-average-molecular weight of 1,000 to 20,000, and (B) at least one selected from the group consisting of a water-soluble vitamin, an amino acid, and a salt thereof.
[2] The ophthalmic composition according to [1], wherein the content of component (A) is 0.001 to 1 w/v% based on the total amount of the ophthalmic composition.
[3] The ophthalmic composition according to [1] or [2], which does not contain a nonionic surfactant.
[4] The ophthalmic composition according to any one of [1] to [3], which is for soft contact lenses.

### Advantageous Effects of Invention

According to the present invention, it is possible to provide a novel ophthalmic composition containing sodium chondroitin sulfate having a weight-average-molecular weight of 1,000 to 20,000.

### Description of Embodiments

Hereinafter, embodiments for carrying out the present invention will be described in detail. However, the present invention is not limited to the following embodiments.
In this specification, unless otherwise specified, the unit of content "%" means "w/v%" and is synonymous with "g/100 mL".

[(A) At least one selected from the group consisting of chondroitin sulfate and a salt thereof, having a weight-average-molecular weight of 1,000 to 20,000]

The ophthalmic composition according to the present embodiment contains at least one selected from the group consisting of chondroitin sulfate and a salt thereof, having a weight-average-molecular weight of 1,000 to 20,000 (also simply referred to as "component (A)").

The chondroitin sulfate and the salt thereof are not particularly limited as long as they are pharmaceutically, pharmacologically (medicinally) or physiologically acceptable.

Examples of the salt of chondroitin sulfate include an alkali metal salt and an alkaline earth metal salt. Examples of the alkali metal salt include a sodium salt and a potassium salt. Examples of the alkaline earth metal salt include a magnesium salt and a calcium salt.

As the chondroitin sulfate and the salt thereof, chondroitin sulfate and an alkali metal salt of chondroitin sulfate are preferable, chondroitin sulfate and sodium chondroitin sulfate are more preferable, and sodium chondroitin sulfate is even more preferable.

The chondroitin sulfate and the salt thereof may be a natural product or a synthetic product, but generally, chondroitin sulfate and a salt thereof derived from natural animals (preferably mammals, fish, mollusks, etc.; more preferably cows, sharks, squids, rays, etc.), chondroitin sulfate and a salt thereof derived from mushrooms such as wood ear mushrooms, and chondroitin sulfate and a salt thereof derived from algae such as seaweed are preferably used, and chondroitin sulfate and a salt thereof derived from sharks and/or rays are more preferably used. In addition, chondroitin sulfate and a salt thereof produced by fermentation using microorganisms such as lactic acid bacteria, Escherichia coli, and yeast are preferably used.

Commercially available products can also be used as the chondroitin sulfate and the salt thereof. The chondroitin sulfate and the salt thereof may be used alone as one type, or two or more types may be used in combination. Further, as the chondroitin sulfate and the salt thereof, chondroitin sulfate and a salt thereof, having a weight-average-molecular weight of 1,000 to 20,000 as defined in this specification can be used in combination with chondroitin sulfate and a salt thereof, having a weight-average-molecular weight outside the range defined in this specification. For example, sodium chondroitin sulfate having a weight-average-molecular weight of 16,000 can be used in combination with sodium chondroitin sulfate having a weight-average-molecular weight of 25,000. When using a combination of chondroitin sulfate and a salt thereof, having a weight-average-molecular weight of 1,000 to 20,000 as defined in this specification and chondroitin sulfate and a salt thereof, having a weight-average-molecular weight outside the range defined in this specification, it is sufficient that chondroitin sulfate and a salt thereof, having a weight-average-molecular weight of 1,000 to 20,000 are included as a raw material.

In this specification, the "weight-average-molecular weight" can be determined by using gel permeation chromatography with an online connection of a multi-angle light scattering detector (MALS detector) and a differential refractive index detector (RI detector). Specifically, the following conditions are presented.

<Standard sample preparation>

A sample prepared by adding 10 mL of a 0.1 M aqueous sodium nitrate solution to 5 mg of chondroitin sulfate or a salt thereof, gently stirring at room temperature, and completely dissolving it.

<Measurement conditions for weight-average-molecular weight>

    Apparatus: Gel permeation chromatograph-multi-angle light scattering photometer
    Detector: Differential refractive index detector (Optilab rEX, manufactured by Wyatt Technology)
    Multi-angle light scattering detector (DAWN HELEOS, manufactured by Wyatt Technology)
    Column: Two Shodex OHpak SB-806M HQ columns ($\varphi$7.8 mm $\times$ 30 cm, manufactured by Showa Denko)
    Solvent: 0.1 M aqueous sodium nitrate solution
    Flow rate: 0.7 mL/min
    Column temperature: 23°C
    Detector temperature: 23°C
    Injection volume: 0.2 mL
    Data processing: Data processing system (ASTRA) manufactured by Wyatt Technology

The weight-average-molecular weight of the chondroitin sulfate and the salt thereof calculated by the above method is not particularly limited as long as it is in the range of 1,000 to 20,000. Examples of the lower limit of the weight-average-molecular weight include 1,000 or more, 2,000 or more, 5,000 or more, 10,000 or more, 11,000 or more, 12,000 or more, and 14,000 or more. Examples of the upper limit of the weight-average-molecular weight include 20,000 or less, 19,500 or less, 19,000 or less, 18,000 or less, 17,000 or less, 15,000 or less, 14,000 or less, and 13,000 or less. Examples of the range of the weight-average-molecular weight include 1,000 to 20,000, 2,000 to 19,500, 5,000 to 19,000, 12,000 to 18,000, and 14,000 to 17,000. The range of the weight-average-molecular weight may also be 10,000 to 14,000.

The content of component (A) in the ophthalmic composition according to the present embodiment is not particularly limited, and is appropriately set according to the type and content of other blended components, the use and formulation form of the ophthalmic composition, and the like. From the viewpoint of more remarkably exhibiting the effects of the present invention, the content of component (A) is, for example, preferably 0.001 to 1 w/v%, more preferably 0.005 to 0.75 w/v%, and even more preferably 0.01 to 0.5 w/v%, based on the total amount of the ophthalmic composition. The content of component (A) may also be 0.05 to 2 w/v%, 0.1 to 1 w/v%, or 0.5 to 1 w/v%, and may be 0.5 w/v% or 1 w/v%.

[(B) At least one selected from the group consisting of a water-soluble vitamin, an amino acid, and a salt thereof]

The ophthalmic composition according to the present embodiment contains at least one selected from the group consisting of a water-soluble vitamin, an amino acid, and a salt thereof (also simply referred to as "component (B)"). The water-soluble vitamin, the amino acid, or the salt thereof is not particularly limited as long as it is pharmaceutically, pharmacologically (medicinally) or physiologically acceptable.

Examples of the water-soluble vitamin include B vitamins such as flavin adenine dinucleotide and a salt thereof (e.g., sodium flavin adenine dinucleotide), cobalamins (e.g., cyanocobalamin, methylcobalamin), pantothenic acid and a salt thereof (e.g., sodium pantothenate, potassium pantothenate, calcium pantothenate, magnesium pantothenate, etc.), panthenol, pyridoxine or a salt thereof (e.g., pyridoxine hydrochloride), and pyridoxal and a salt thereof (e.g., pyridoxal phosphate); and vitamin C such as ascorbic acid and a salt thereof (e.g., sodium ascorbate). Among the water-soluble vitamins, from the viewpoint of further enhancing the effects of the present invention, B vitamins are preferable, panthenol, pyridoxine or a salt thereof, flavin adenine dinucleotide or a salt thereof, and cyanocobalamin are more preferable, and panthenol and pyridoxine or a salt thereof are even more preferable.

Commercially available products can also be used as the water-soluble vitamin. The water-soluble vitamin may be used alone as one type, or two or more types may be used in combination.

Examples of the amino acid include aspartic acid, glutamic acid, asparagine, glutamine, arginine, lysine, glycine, alanine, γ-aminobutyric acid, γ-aminovaleric acid, trimethylglycine, epsilon-aminocaproic acid, aminoethylsulfonic acid (taurine), and the like. Examples of the salt of the amino acid include a salt with an inorganic acid (e.g., salts with hydrochloric acid, hydrobromic acid, sulfuric acid, nitric acid, phosphoric acid, etc.), a salt with an organic acid (e.g., salts with acetic acid, succinic acid, fumaric acid, maleic acid, tartaric acid, citric acid, lactic acid, stearic acid, benzoic acid, methanesulfonic acid (mesylic acid), ethanesulfonic acid, p-toluenesulfonic acid, etc.), a salt with an inorganic base (e.g., alkali metal salts such as a sodium salt and a potassium salt; alkaline earth metal salts such as a calcium salt and a magnesium salt; an aluminum salt; an ammonium salt), and salts with an organic base (e.g., salts with diethylamine, diethanolamine, meglumine, N,N-dibenzylethylenediamine, etc.). Commercially available products can also be used as the amino acid and the salt thereof. The amino acid and the salt thereof may be any of an L-form, a D-form, and a DL-form. Among the amino acids and salts thereof, from the viewpoint of further enhancing the effects of the present invention, aspartic acid or a salt thereof, epsilon-aminocaproic acid, and aminoethylsulfonic acid are preferable, and aminoethylsulfonic acid is more preferable.

The content of component (B) in the ophthalmic composition according to the present embodiment is not particularly limited, and is appropriately set according to the type of component (B), the type and content of other blended components, the formulation form, and the like. From the viewpoint of more remarkably exhibiting the effects of the present invention, for example, the total content of component (B) is preferably 0.0001 to 5 w/v%, more preferably 0.0005 to 4 w/v%, and even more preferably 0.001 to 3 w/v%, based on the total amount of the ophthalmic composition according to the present embodiment. The total content of component (B) may also be 0.005 to 1 w/v% or 0.01 to 0.1 w/v%.

The content proportion of component (B) to component (A) in the ophthalmic composition according to the present embodiment is not particularly limited, and is appropriately set according to the types of component (A) and component (B), the type and content of other blended components, the use and formulation form of the ophthalmic composition, and the like. From the viewpoint of further enhancing the effects of the present invention, for example, the content proportion of component (B) to component (A) is such that the total content of component (B) is preferably 0.0001 to 5000 parts by mass, more preferably 0.0005 to 4000 parts by mass, and even more preferably 0.001 to 3000 parts by mass, with respect to 1 part by mass of the total content of component (A) included in the ophthalmic composition according to the present embodiment. The total content of component (B) may also be 0.001 to 20 parts by mass, 0.01 to 5 parts by mass, 0.01 to 1 part by mass, or 0.02 to 2 parts by mass, with respect to 1 part by mass of the total content of component (A).

When component (B) is a water-soluble vitamin, from the viewpoint of more remarkably exhibiting the effects of the present invention, for example, the total content of the water-soluble vitamin is preferably 0.0001 to 0.5 w/v%, more preferably 0.0005 to 0.3 w/v%, and even more preferably 0.001 to 0.2 w/v%, based on the total amount of the ophthalmic composition according to the present embodiment. When component (B) is a water-soluble vitamin, the total content of the water-soluble vitamin may also be 0.01 to 0.1 w/v%.

When component (B) is a water-soluble vitamin, from the viewpoint of further enhancing the effects of the present invention, for example, the content proportion of the water-soluble vitamin to component (A) is such that the total content of the water-soluble vitamin is preferably 0.0001 to 500 parts by mass, more preferably 0.0005 to 300 parts by mass, and even more preferably 0.001 to 200 parts by mass, with respect to 1 part by mass of the total content of component (A) included in the ophthalmic composition according to the present embodiment. When component (B) is a water-soluble vitamin, the total content of the water-soluble vitamin may also be 0.001 to 20 parts by mass, 0.01 to 5 parts by mass, 0.01 to 1 part by mass, or 0.02 to 2 parts by mass, with respect to 1 part by mass of the total content of component (A).

When component (B) is an amino acid and a salt thereof, from the viewpoint of more remarkably exhibiting the effects of the present invention, for example, the total content of the amino acid and the salt thereof is preferably 0.0001 to 5 w/v%, more preferably 0.001 to 4 w/v%, and even more preferably 0.05 to 3 w/v%, based on the total amount of the ophthalmic composition according to the present embodiment. When component (B) is an amino acid and a salt thereof, the total content of the amino acid and the salt thereof may also be 0.1 to 2 w/v% or 0.2 to 1 w/v%. Furthermore, when component (B) is aminoethylsulfonic acid, the content of aminoethylsulfonic acid is preferably 0.1 to 1 w/v%.

When component (B) is an amino acid and a salt thereof, from the viewpoint of further enhancing the effects of the present invention, for example, the content proportion of the amino acid and the salt thereof to component (A) is such that the total content of the amino acid and the salt thereof is preferably 0.0001 to 5000 parts by mass, more preferably 0.001 to 4000 parts by mass, and even more preferably 0.05 to 3000 parts by mass, with respect to 1 part by mass of the total content of component (A) included in the ophthalmic composition according to the present embodiment. When component (B) is an amino acid and a salt thereof, the total content of the amino acid and the salt thereof may also be 0.001 to 20 parts by mass, 0.01 to 5 parts by mass, 0.01 to 1 part by mass, or 0.02 to 2 parts by mass, with respect to 1 part by mass of the total content of component (A).

[Buffer]

The ophthalmic composition according to the present embodiment preferably further contains a buffer. When the ophthalmic composition further contains a buffer, the effects of the present invention are more remarkably exhibited. The buffer is not particularly limited as long as it is pharmaceutically, pharmacologically (medicinally) or physiologically acceptable. Examples of the buffer include an inorganic buffer, which is a buffer derived from an inorganic acid, and an organic buffer, which is a buffer derived from an organic acid or an organic base.

Examples of the inorganic buffer include a borate buffer, a phosphate buffer, a carbonate buffer, and the like. Examples of the borate buffer include boric acid or a salt thereof (e.g., an alkali metal borate, an alkaline earth metal borate). Examples of the phosphate buffer include phosphoric acid or a salt thereof (e.g., an alkali metal phosphate, an alkaline earth metal phosphate). Examples of the carbonate buffer include carbonic acid or a salt thereof (e.g., an alkali metal carbonate, an alkaline earth metal carbonate). A hydrate of a borate, a phosphate, or a carbonate may also be used as the borate buffer, the phosphate buffer, or the carbonate buffer. More specific examples include, as the borate buffer, boric acid or a salt thereof (e.g., sodium borate, potassium tetraborate, potassium metaborate, ammonium borate, borax); as the phosphate buffer, phosphoric acid or a salt thereof (e.g., disodium hydrogen phosphate, sodium dihydrogen phosphate, potassium dihydrogen phosphate, trisodium phosphate, tripotassium phosphate, calcium monohydrogen phosphate, calcium dihydrogen phosphate); and as the carbonate buffer, carbonic acid or a salt thereof (e.g., sodium bicarbonate, sodium carbonate, ammonium carbonate, potassium carbonate, calcium carbonate, potassium bicarbonate, magnesium carbonate).

Examples of the organic buffer include a citrate buffer, an acetate buffer, a lactate buffer, a succinate buffer, a tris buffer, an AMPD buffer, and the like. Examples of the citrate buffer include citric acid or a salt thereof (e.g., an alkali metal citrate, an alkaline earth metal citrate). Examples of the acetate buffer include acetic acid or a salt thereof (e.g., an alkali metal acetate, an alkaline earth metal acetate). Examples of the lactate buffer include lactic acid or a salt thereof (e.g., an alkali metal lactate, an alkaline earth metal lactate). Examples of the succinate buffer include succinic acid or a salt thereof (e.g., an alkali metal succinate). A hydrate of a citrate, an acetate, a lactate, or a succinate may also be used as the citrate buffer, the acetate buffer, the lactate buffer, or the succinate buffer. More specific examples include, as the citrate buffer, citric acid or a salt thereof (e.g., sodium citrate, potassium citrate, calcium citrate, sodium dihydrogen citrate, disodium citrate); as the acetate buffer, acetic acid or a salt thereof (e.g., ammonium acetate, sodium acetate, potassium acetate, calcium acetate); as the lactate buffer, lactic acid or a salt thereof (e.g., sodium lactate, potassium lactate, calcium lactate); and as the succinate buffer, succinic acid or a salt thereof (e.g., monosodium succinate, disodium succinate). Examples of the tris buffer include trometamol or a salt thereof (e.g., trometamol hydrochloride). Examples of the AMPD buffer include 2-amino-2-methyl-1,3-propanediol or a salt thereof.

As the buffer, a borate buffer (e.g., a combination of boric acid and borax), boric acid and a salt thereof are more preferable, and a combination of boric acid and borax is even more preferable.

Commercially available products may be used as the buffer. The buffer may be used alone as one type, or two or more types may be used in combination.

The content of the buffer in the ophthalmic composition according to the present embodiment is not particularly limited, and is appropriately set according to the type of the buffer, the type and content of other blended components, the use and formulation form of the ophthalmic composition, and the like. From the viewpoint of more remarkably exhibiting the effects of the present invention, for example, the total content of the buffer is preferably 0.01 to 4 w/v%, more preferably 0.05 to 3 w/v%, and even more preferably 0.1 to 2 w/v%, based on the total amount of the ophthalmic composition.

The content proportion of the buffer to component (A) in the ophthalmic composition according to the present embodiment is not particularly limited, and is appropriately set according to the types of component (A) and the buffer, the type and content of other blended components, the use and formulation form of the ophthalmic composition, and the like. From the viewpoint of further enhancing the effects of the present invention, for example, the content proportion of the buffer to component (A) is such that the total content of the buffer is preferably 0.01 to 4000 parts by mass, more preferably 0.05 to 3000 parts by mass, and even more preferably 0.1 to 2000 parts by mass, with respect to 1 part by mass of the total content of component (A) included in the ophthalmic composition according to the present embodiment.

The pH of the ophthalmic composition according to the present embodiment is not particularly limited as long as it is within a pharmaceutically, pharmacologically (medicinally) or physiologically acceptable range. The pH of the ophthalmic composition according to the present embodiment may be, for example, 4.0 to 9.5, preferably 4.0 to 9.0, more preferably 4.5 to 9.0, even more preferably 4.5 to 8.5, still more preferably 5.0 to 8.5, and particularly preferably 5.0 to 8.0.

The ophthalmic composition according to the present embodiment may be adjusted to an osmotic pressure ratio within a biologically acceptable range, if necessary. An appropriate osmotic pressure ratio can be appropriately set according to the use, formulation form, method of use, etc., of the ophthalmic composition; for example, it may be 0.4 to 5.0, preferably 0.6 to 3.0, more preferably 0.8 to 2.2, and even more preferably 0.8 to 2.0. The osmotic pressure ratio is the ratio of the osmotic pressure of a sample to 286 mOsm (the osmotic pressure of a 0.9 w/v% aqueous sodium chloride solution), based on the 17th revised Japanese Pharmacopoeia, and the osmotic pressure is measured with reference to the Osmometry (freezing point depression method) described in the Japanese Pharmacopoeia. The standard solution for osmotic pressure ratio measurement (0.9 w/v% aqueous sodium chloride solution) can be prepared by drying sodium chloride (Japanese Pharmacopoeia Standard Reagent) at 500 to 650°C for 40 to 50 minutes, allowing it to cool in a desiccator (silica gel), accurately weighing 0.900 g thereof, and dissolving it in purified water to make exactly 100 mL, or a commercially available standard solution for osmotic pressure ratio measurement (0.9 w/v% aqueous sodium chloride solution) can be used.

The viscosity of the ophthalmic composition according to the present embodiment is not particularly limited as long as it is within a pharmaceutically, pharmacologically (medicinally) or physiologically acceptable range. As for the viscosity of the ophthalmic composition according to the present embodiment, for example, the viscosity at 20°C measured with a rotational viscometer (TV-20 viscometer, manufactured by Toki Sangyo Co., Ltd., rotor; 1°34' x R24) is preferably 1 to 10000 mPa·s, more preferably 1 to 8000 mPa·s, even more preferably 1 to 1000 mPa·s, still more preferably 1 to 100 mPa·s, particularly preferably 1 to 20 mPa·s, and most preferably 1.5 to 10 mPa·s.

The ophthalmic composition according to the present embodiment may contain an appropriate amount of a component selected from various pharmacologically active components and physiologically active components in combination with the above components, as long as the effects of the present invention are not impaired. The component is not particularly limited, and for example, active components in ophthalmic drugs described in the "Over-the-Counter Drug Manufacturing and Sales Approval Standards, 2017 Edition" (supervised by general incorporated association, the Society of Regulatory Science) can be exemplified. Specific examples of components used in ophthalmic drugs include the following components.

Antiallergic agents: for example, sodium cromoglicate, tranilast, pemirolast potassium, acitazanolast, amlexanox, ibudilast, etc.

Antihistamines: for example, chlorpheniramine or a salt thereof (e.g., chlorpheniramine maleate), diphenhydramine or a salt thereof (e.g., diphenhydramine hydrochloride), iproheptine or a salt thereof (e.g., iproheptine hydrochloride), levocabastine or a salt thereof (e.g., levocabastine hydrochloride), ketotifen or a salt thereof (e.g., ketotifen fumarate), pemirolast potassium, olopatadine or a salt thereof (e.g., olopatadine hydrochloride), etc.

Antiphlogistic agents: for example, methyl salicylate, glycol salicylate, allantoin, tranexamic acid, lysozyme, lysozyme chloride, indomethacin, pranoprofen, ibuprofen, ibuprofen piconol, ketoprofen, felbinac, bendazac, piroxicam, bufexamac, butyl flufenamate, epsilon-aminocaproic acid, berberine chloride, berberine sulfate, sodium azulene sulfonate, glycyrrhizic acid or a salt thereof (e.g., dipotassium glycyrrhizinate, monoammonium glycyrrhizinate), etc.

Steroids: for example, fluticasone propionate, fluticasone furoate, mometasone furoate, beclomethasone propionate, flunisolide, etc.

Decongestants: for example, tetrahydrozoline hydrochloride, tetrahydrozoline nitrate, naphazoline hydrochloride, naphazoline nitrate, epinephrine, epinephrine hydrochloride, ephedrine hydrochloride, phenylephrine hydrochloride, dl-methylephedrine hydrochloride, etc.

Eye muscle regulating agents: for example, cholinesterase inhibitors having an active center similar to acetylcholine, specifically neostigmine methyl sulfate, tropicamide, helenien, atropine sulfate, pilocarpine hydrochloride, etc.
Fat-soluble vitamins: for example, retinol acetate, retinol palmitate, tocopherol acetate, etc.
Others: for example, sulfamethoxazole, sulfisoxazole, sulfisomidine, and salts thereof, etc.

The ophthalmic composition according to the present embodiment may contain an appropriate amount of one or more various additives, appropriately selected according to its use and formulation form in accordance with conventional methods, as long as the effects of the present invention are not impaired. Examples of such additives include various additives described in the "Pharmaceutical Excipients Directory 2016" (edited by the Japan Pharmaceutical Excipients Association). The following additives are mentioned as representative components.

Carriers: for example, aqueous solvents such as water and hydrous ethanol.
Chelating agents: for example, ethylenediaminediacetic acid (EDDA), ethylenediaminetriacetic acid, ethylenediaminetetraacetic acid (EDTA), N-(2-hydroxyethyl)ethylenediaminetriacetic acid (HEDTA), diethylenetriaminepentaacetic acid (DTPA), etc.
Bases: for example, octyldodecanol, titanium oxide, potassium bromide, Plastibase, etc.
pH regulating agents: for example, hydrochloric acid, acetic acid, sodium hydroxide, potassium hydroxide, calcium hydroxide, magnesium hydroxide, triethanolamine, monoethanolamine, diisopropanolamine, etc. Thickeners: for example, cellulose-based polymer compounds such as methylcellulose, ethylcellulose, hydroxypropyl methylcellulose, hydroxyethyl cellulose, and sodium carboxymethylcellulose; polyvinyl-based polymer compounds such as polyvinylpyrrolidone and polyvinyl alcohol; carboxyvinyl polymer; guar gum; hydroxypropyl guar gum; gum arabic; karaya gum; xanthan gum; agar; alginic acid and a salt thereof (e.g., sodium salt); mucopolysaccharides such as heparin-like substances, heparin, heparin sulfate, heparan sulfate, heparinoids, hyaluronic acid and a salt thereof (e.g., sodium salt); starch; chitin and a derivative thereof; chitosan and a derivative thereof; carrageenan; monosaccharides such as glucose, etc.
Stabilizers: for example, edetic acid, edetates (disodium edetate, calcium disodium edetate, trisodium edetate, tetrasodium edetate), sodium formaldehyde sulfoxylate (rongalite), aluminum monostearate, glyceryl monostearate, cyclodextrin, monoethanolamine, dibutylhydroxytoluene, sodium bisulfite, sodium pyrosulfite, etc.
Preservatives: for example, alkyl polyaminoethylglycine-type quaternary ammonium salts (e.g., benzalkonium chloride, benzethonium chloride, etc.), chlorhexidine gluconate, polidronium chloride, zinc chloride, sodium benzoate, ethanol, chlorobutanol, sorbic acid, potassium sorbate, sodium dehydroacetate, methyl paraoxybenzoate, ethyl paraoxybenzoate, propyl paraoxybenzoate, butyl paraoxybenzoate, oxyquinoline sulfate, phenethyl alcohol, benzyl alcohol, biguanide compounds (specifically, polyhexanide hydrochloride (polyhexamethylene biguanide), alexidine, etc.), Glokill (trade name, manufactured by Rhodia), etc.
Isotonic agents: for example, potassium chloride, calcium chloride, sodium chloride, magnesium chloride, potassium acetate, sodium acetate, sodium bicarbonate, sodium carbonate, sodium thiosulfate, magnesium sulfate, glycerin, propylene glycol, sodium bisulfite, sodium sulfite, etc. Sugar alcohols: for example, xylitol, sorbitol, mannitol, glycerin, etc. These may be any of d-form, l-form, or dl-form.

The ophthalmic composition according to the present embodiment preferably does not contain POE sorbitan monolaurate, more preferably does not contain POE sorbitan monolaurate and polyoxyethylene hydrogenated castor oil, and even more preferably does not contain a nonionic surfactant, because the effects of the present invention can be more remarkably exhibited. Examples of the nonionic surfactant include POE sorbitan fatty acid esters such as POE (20) sorbitan monolaurate (polysorbate 20), POE (20) sorbitan monopalmitate (polysorbate 40), POE (20) sorbitan monostearate (polysorbate 60), POE (20) sorbitan tristearate (polysorbate 65), and POE (20) sorbitan monooleate (polysorbate 80); POE/POP glycols such as poloxamer 407, poloxamer 235, poloxamer 188, poloxamer 403, poloxamer 237, and poloxamer 124; POE hydrogenated castor oils such as POE hydrogenated castor oil 40, POE hydrogenated castor oil 50, POE hydrogenated castor oil 60, and POE hydrogenated castor oil 80; POE castor oils such as POE castor oil 3, POE castor oil 4, POE castor oil 6, POE castor oil 7, POE castor oil 10, POE castor oil 13.5, POE castor oil 17, POE castor oil 20, POE castor oil 25, POE castor oil 30, POE castor oil 35, and POE castor oil 50; polyethylene glycol monostearates such as polyethylene glycol (2 E.O.) monostearate, polyethylene glycol (4 E.O.) monostearate, polyethylene glycol (9 E.O.) monostearate, polyethylene glycol (10 E.O.) monostearate, polyethylene glycol (23 E.O.) monostearate, polyethylene glycol (25 E.O.) monostearate, polyethylene glycol (32 E.O.) monostearate, polyethylene glycol (40 E.O.) monostearate (polyoxyl 40 stearate), polyethylene glycol (45 E.O.) monostearate, polyethylene glycol (55 E.O.) monostearate, polyethylene glycol (75 E.O.) monostearate, and polyethylene glycol (140 E.O.) monostearate; POE alkyl ethers such as POE (9) lauryl ether; POE-POP alkyl ethers such as POE (20) POP (4) cetyl ether; and POE alkylphenyl ethers such as POE (10) nonylphenyl ether. In the compounds exemplified above, POE indicates polyoxyethylene, POP indicates polyoxypropylene, and the number in parentheses indicates the number of added moles.

The ophthalmic composition according to the present embodiment preferably does not contain a terpenoid, because the effects of the present invention can be more remarkably exhibited. Examples of the terpenoid include menthol, camphor, borneol, and geraniol.

When the ophthalmic composition according to the present embodiment contains water, from the viewpoint of more remarkably exhibiting the effects of the present invention, for example, the content of water is preferably 80 w/v% or more and less than 100 w/v%, more preferably 85 w/v% or more and 99.5 w/v% or less, and even more preferably 90 w/v% or more and 99.2 w/v% or less, based on the total amount of the ophthalmic composition.

The water used in the ophthalmic composition according to the present embodiment may be any as long as it is pharmaceutically, pharmacologically (medicinally) or physiologically acceptable. Examples of such water include distilled water, tap water, purified water, sterile purified water, water for injection, and distilled water for injection. Their definitions are based on the 17th revised Japanese Pharmacopoeia.

The ophthalmic composition according to the present embodiment can be prepared by adding and mixing desired amounts of component (A), component (B), and other components as necessary to achieve desired concentrations. For example, it can be prepared by dissolving or dispersing these components in purified water, adjusting to a predetermined pH and osmotic pressure, and performing a sterilization treatment such as sterile filtration.

The ophthalmic composition according to the present embodiment can take various formulation forms depending on the purpose. Examples of the formulation form include a liquid preparation, a gel preparation, a semi-solid preparation (ointment, etc.), and the like.

The ophthalmic composition according to the present embodiment can be used, for example, as an eye drop (also referred to as an eye lotion or an ophthalmic preparation; eye drops also include those that can be instilled while wearing contact lenses), artificial tears, an eyewash (also referred to as an eyewash liquid or an eyewash preparation; eyewashes also include those that can be used for eye washing while wearing contact lenses), or a contact lens composition [contact lens wearing liquid, contact lens care composition (contact lens disinfectant, contact lens preservative, contact lens cleaning agent, contact lens cleaning preservative), contact lens packaging liquid, etc.]. The "contact lens" includes hard contact lenses and soft contact lenses (including both ionic and non-ionic, and including both silicone hydrogel contact lenses and non-silicone hydrogel contact lenses).

The ophthalmic composition according to the present embodiment is preferably an eye drop (including an eye drop that can be instilled while wearing contact lenses), because the effects of the present invention can be more remarkably exhibited. When the ophthalmic composition according to the present embodiment is an eye drop, its usage and dosage are not particularly limited as long as they are effective and have few side effects; for example, in the case of adults (15 years or older) and children of 7 years or older, a method of use can be exemplified in which 1 to 3 drops, 1 to 2 drops, or 2 to 3 drops are instilled 2 to 4 times a day, or 5 to 6 times a day.

The ophthalmic composition according to the present embodiment is provided after being stored in an arbitrary container. The container for storing the ophthalmic composition according to the present embodiment is not particularly limited, and may be made of glass or plastic, for example. It is preferably made of plastic. Examples of the plastic include polyethylene terephthalate (PET), polyarylate (PAR), polyethylene naphthalate (PEN), polycarbonate (PC), polyethylene (PE), polypropylene (PP), polyimide (PI), cyclic olefin copolymer (COC), and copolymers of monomers constituting these, as well as mixtures of two or more of these. Polyethylene terephthalate (PET) is preferable. The container for storing the ophthalmic composition according to the present embodiment may be a transparent container that allows the inside of the container to be visually inspected, or an opaque container that makes it difficult to visually inspect the inside of the container. A transparent container is preferable. Here, "transparent container" includes both colorless transparent containers and colored transparent containers.

A nozzle may be attached to the container storing the ophthalmic composition according to the present embodiment. The material of the nozzle is not particularly limited, and may be made of glass or plastic, for example. It is preferably made of plastic. Examples of the plastic include polybutylene terephthalate (PBT), polyethylene (PE), polypropylene (PP), polyethylene terephthalate (PET), polyethylene naphthalate (PEN), and copolymers of monomers constituting these, as well as mixtures of two or more of these. As the material of the nozzle, from the viewpoint of further enhancing the effects of the present invention, polypropylene (PP), polyethylene (PE), polyethylene terephthalate (PET), polybutylene terephthalate (PBT), and polyethylene naphthalate (PEN) are preferable, and polyethylene (PE) is more preferable.

The container storing the ophthalmic composition according to the present embodiment may be a multi-dose type that stores a quantity for multiple uses, or a unit-dose type that stores a quantity for a single use.

The ophthalmic composition according to the present embodiment is preferably filled in a container having an internal volume of 4 to 30 mL, more preferably filled in a container having an internal volume of 5 to 20 mL, even more preferably filled in a container having an internal volume of 10 to 18 mL, and still more preferably filled in a container having an internal volume of 15 to 18 mL. It may also be filled in a container having an internal volume of 0.1 to 3 mL, or filled in a container having an internal volume of 0.2 to 1 mL.

As shown in the Examples described later, the ophthalmic composition according to the present embodiment has an effect of significantly reducing the elastic modulus of soft contact lenses while containing sodium chondroitin sulfate having a weight-average-molecular weight of 1,000 to 20,000, and is therefore preferably an ophthalmic composition for soft contact lenses. When the elastic modulus of a soft contact lens is reduced, the soft contact lens becomes soft, which can more effectively reduce discomfort such as a feeling of rumbling in the eyes and a feeling of stiffness in the eyes during and/or upon wearing the soft contact lens, and discomfort due to poor wearing sensation. Further, as one embodiment of the present invention, a method is provided for imparting to an ophthalmic composition an effect of reducing the elastic modulus of a soft contact lens, which includes incorporating (B) at least one selected from the group consisting of a water-soluble vitamin, an amino acid, and a salt thereof into an ophthalmic composition containing (A) at least one selected from the group consisting of chondroitin sulfate and a salt thereof, having a weight-average-molecular weight of 1,000 to 20,000.

In the above embodiment, the rate of reducing the elastic modulus of the soft contact lens (hereinafter also referred to as "reduction rate of elastic modulus") is not particularly limited, but it is preferable that the reduction rate of the elastic modulus of the soft contact lens is 10 to 50%. Here, the reduction rate of the elastic modulus of the soft contact lens in the present embodiment is calculated by the following formula. In this specification, the "elastic modulus of a soft contact lens" means a value measured by using an MCR302 rheometer manufactured by Anton Paar and using a parallel plate PP12/P12.

Reduction rate of elastic modulus (%) = ((Elastic modulus of comparative composition (Pa)) - (Elastic modulus of agent according to the present embodiment (Pa))) / (Elastic modulus of comparative composition (Pa)) × 100　　　　　[Formula]

Here, the comparative composition is a composition having the same composition as the ophthalmic composition according to the present embodiment, except that it does not contain at least one selected from the group consisting of (B) a water-soluble vitamin, an amino acid, and a salt thereof.

**Examples**

Hereinafter, the present invention will be specifically described based on test examples, but the present invention is not limited to these.

[Preparation of Test Solutions]

The test solutions shown in Table 1 were prepared according to a conventional method. The unit for each component in Table 1 is g/100 mL. The sodium chondroitin sulfate used in the following test examples is as follows.

Sodium chondroitin sulfate

Weight-average-molecular weight of about 16,000: Maruha Nichiro Corporation: Japanese Pharmaceutical Codex sodium chondroitin sulfate

[Test Example 1: Evaluation of Change in Elastic Modulus]

4 mL of physiological saline solution (manufactured by Otsuka Pharmaceutical Factory, Inc.) was dispensed into each well of a 12-well plate (BD Falcon, No. 35-3043), and one contact lens (PRECISION1: manufactured by Alcon) was immersed in each well and left to stand at room temperature for 4 hours or more. 4 mL of each test solution shown in Table 1 prepared above was dispensed into each well of another 12-well plate (BD Falcon, No. 35-3043), and one contact lens, from which moisture had been lightly wiped with a lint-free paper, was immersed in each well and left to stand at 34°C for 24 hours.

Using an MCR302 rheometer (manufactured by Anton Paar), with a parallel plate PP12/P12 (diameter 12 mm, grid pattern; 1 x 0.5, product number: 23935) and a cap plate P-PTD200/80-77/SS/P2 (grid pattern, product number: 7894), the measurement temperature was set to 20°C, the frequency to 1 Hz, and the measurement time to "no time setting" mode, and the shear stress (Pa) for a strain of 0.01 to 1% was measured. Specifically, 0.5 mL of physiological saline solution was dropped onto the cap plate, the convex surface of a contact lens from which moisture had been wiped off was placed on it (the convex surface of the lens facing the cap plate side), and 0.5 mL of physiological saline solution was dropped onto the contact lens. The parallel plate was lowered toward the cap plate, the position was set so that the contact lens was sandwiched at a distance of 0.1 mm between the two plates, and the measurement of shear stress (Pa) was started. The measurement intervals were 5 points equally spaced in the strain range of 0.01 to 0.1% and 5 points equally spaced in the strain range of 0.1 to 1%. The elastic modulus (Pa) was calculated using [Formula 1] with 0.0325%, which is the second measurement point in the strain range of 0.01 to 0.1%, and 1% in the measurement interval of the strain range of 0.1 to 1%. The measurement was performed three times, and the average value was taken as the average elastic modulus (Pa).

Elastic modulus (Pa) = Shear stress / Strain = ((Shear stress at 1% strain) - (Shear stress at 0.0325% strain)) / (1 - 0.0325) [Formula 1]

Using the average elastic modulus (Pa) in Examples 1 to 3, the reduction rate of elastic modulus (%) relative to the corresponding reference example was calculated according to [Formula 2].

Reduction rate of elastic modulus relative to corresponding reference example (%) = ((Average elastic modulus of corresponding reference example (Pa) - Average elastic modulus of example (Pa)) / Average elastic modulus of corresponding reference example (Pa)) $\times$ 100 [Formula 2]

The corresponding reference example for Examples 1 to 3 is Reference Example 1.

[Table 1]

| | Example 1 | Example 2 | Example 3 | Reference Example 1 |
|---|---|---|---|---|
| Sodium chondroitin sulfate (Weight-average-molecular weight: 16,000) | 0.5 | 0.5 | 0.5 | 0.5 |
| Pyridoxine hydrochloride | 0.1 | - | - | - |
| Panthenol | - | 0.1 | - | - |
| Aminoethylsulfonic acid | - | - | 1.0 | - |
| Boric acid | 1.2 | 1.2 | 1.2 | 1.2 |
| Borax | 0.1 | 0.1 | 0.1 | 0.1 |
| Purified water | Residual amount | Residual amount | Residual amount | Residual amount |
| Total amount | 100 mL | 100 mL | 100 mL | 100 mL |
| pH | 7 | 7 | 7 | 7 |
| Reduction rate of elastic modulus relative to Reference Example 1 (%) | 17.0 | 16.6 | 31.9 | - |

Examples 1 to 3, which contain sodium chondroitin sulfate having a weight-average-molecular weight of 16,000 and a water-soluble vitamin or an amino acid, showed a reduction rate of the elastic modulus of soft contact lenses of 16.6 to 31.9% relative to Reference Example 1, which does not contain a water-soluble vitamin or an amino acid. This indicated that in an ophthalmic composition containing sodium chondroitin sulfate having a weight-average-molecular weight of 1,000 to 20,000, a water-soluble vitamin or an amino acid has an effect of softening hardened soft contact lenses.

[Formulation Examples]

Formulation Examples 1 to 16 are prepared according to the formulations shown in Tables 2 and 3 below. Preparation Examples 1 to 16 were prepared by filling Formulation Examples 1 to 16 into containers made of polyethylene terephthalate and attaching nozzles made of low-density polyethylene. Preparation Examples 17 to 32 were prepared by filling Formulation Examples 1 to 16 into containers made of polyethylene terephthalate and attaching nozzles, in which all wall surfaces that may come into contact with the content liquid when the cap is attached (during storage) are made of polybutylene terephthalate. Preparation Examples 33 to 48 were prepared by filling Formulation Examples 1 to 16 into containers made of polyethylene terephthalate and attaching nozzles, in which a part of the wall surfaces that may come into contact with the content liquid is made of polyethylene terephthalate.
The units in the tables are w/v% unless otherwise specified.

[Table 2]

| Components | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 | Formulation Example 10 |
|---|---|---|---|---|---|---|---|---|---|---|
| Sodium chondroitin sulfate (Weight-average-molecular weight: 16,000) | 0.5 | 1 | 0.1 | 0.25 | 0.1 | 0.5 | 0.25 | 0.4 | 0.2 | 0.15 |
| Sodium flavin adenine dinucleotide | - | - | 0.05 | - | - | - | - | 0.01 | 0.05 | - |
| Cyanocobalamin | - | 0.01 | - | - | - | - | 0.020 | - | - | - |
| Pyridoxine hydrochloride | - | 0.05 | 0.1 | - | 0.01 | 0.08 | - | - | 0.01 | - |
| Panthenol | 0.05 | - | - | 0.1 | - | 0.025 | - | - | - | 0.05 |
| L-Potassium aspartate | 0.5 | 0.5 | 0.5 | - | 0.8 | - | - | 0.1 | 1 | - |
| L-Magnesium/Potassium aspartate | - | - | - | 0.800 | - | - | 1 | - | 2 | - |
| Taurine | 0.5 | 0.5 | 1 | 0.5 | 1 | - | 0.5 | 0.1 | 1 | - |
| epsilon-Aminocaproic acid | - | 1 | 1 | - | 2.5 | - | - | - | 1 | - |
| Tetrahydrozoline hydrochloride | 0.01 | 0.03 | 0.1 | 0.01 | 0.01 | 0.02 | - | - | - | 0.02 |
| Neostigmine methyl sulfate | - | 0.005 | - | 0.005 | 0.005 | - | - | - | - | 0.0025 |
| Allantoin | 0.1 | - | - | - | - | - | 0.1 | - | - | - |
| Dipotassium glycyrrhizinate | - | 0.1 | 0.25 | - | - | 0.25 | 0.1 | - | - | 0.25 |
| Sodium azulene sulfonate | - | - | - | 0.01 | - | - | - | 0.02 | - | - |

| Components | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 | Formulation Example 10 |
|---|---|---|---|---|---|---|---|---|---|---|
| Berberine chloride | - | - | - | - | - | 0.01 | - | 0.01 | - | - |
| Zinc sulfate | - | 0.05 | - | - | - | - | 0.1 | - | - | 0.1 |
| Chlorphen iramine maleate | - | 0.03 | 0.03 | 0.03 | 0.03 | 0.02 | 0.01 | 0.03 | | - |
| Potassium chloride | - | 0.008 | - | - | - | - | - | - | - | - |
| Sodium chloride | - | - | - | - | - | - | 0.1 | - | - | - |
| Benzalkonium chloride | - | 0.01 | - | - | - | - | - | - | - | - |
| Chlorhexi dine gluconate | - | - | - | - | - | - | - | - | 0.005 | - |
| Polyhexanide hydrochloride | lppm | - | lppm | - | - | - | - | - | - | - |
| Chlorobut anol | 0.1 | 0.2 | 0.1 | - | - | - | 0.2 | - | - | - |
| Zinc chloride | - | - | - | - | - | - | - | - | - | 0.001 |
| Sodium edetate | 0.01 | 0.05 | 0.01 | 0.05 | 0.05 | - | 0.01 | 0.01 | 0.05 | 0.05 |
| Boric acid | 1.2 | 0.2 | 0.3 | 0.1 | 0.5 | 0.3 | 0.1 | 1 | 0.4 | 0.1 |
| Borax | 0.3 | 0.1 | 0.05 | 0.025 | - | - | 0.01 | 0.1 | 0.1 | 0.2 |
| Sodium hydrogen phosphate | - | - | - | - | - | - | - | - | - | 0.15 |
| Sodium dihydrogen phosphate | - | - | - | - | - | - | - | - | - | 0.2 |
| Citric acid | - | - | - | - | - | - | - | - | 0.1 | - |
| Trometam ol | - | - | - | - | 0.3 | - | - | - | - | - |
| Propylene glycol | - | - | - | - | 0.2 | - | - | - | - | - |
| Concentrated glycerin | 0.1 | - | - | - | - | - | - | - | - | - |

EP 4 748 383 A1

12

| Components | Formulation Example 1 | Formulation Example 2 | Formulation Example 3 | Formulation Example 4 | Formulation Example 5 | Formulation Example 6 | Formulation Example 7 | Formulation Example 8 | Formulation Example 9 | Formulation Example 10 |
|---|---|---|---|---|---|---|---|---|---|---|
| Sodium hyaluronate | 0.005 | 0.002 | - | - | - | - | - | - | - | 0.01 |
| Dextran | - | - | - | 0.05 | - | - | - | - | - | - |
| L-Arginine | - | - | - | - | - | - | - | - | - | 0.1 |
| Polyvinyl pyrrolidone | - | - | - | - | - | - | 0.1 | - | - | - |
| Hydroxyethyl cellulose | - | - | 0.1 | - | - | - | - | - | - | - |
| Hydroxypropyl methylcellulose | 0.1 | - | - | - | - | - | 0.2 | - | 0.01 | - |
| Hydrochloric acid | Appropriate amout | Appropriate amout | Appropriate amout | Appropriate amout | Appropriate amout | Appropriate amout | Appropriate amout | Appropriate amout | Appropriate amout | Appropriate amout |
| Sodium hydroxide | Appropriate amount | Appropriate amount | Appropriate amount | Appropriate amount | Appropriate amount | Appropriate amount | Appropriate amount | Appropriate amount | Appropriate amount | Appropriate amount |
| Purified water | Residual amout | Residual amout | Residual amout | Residual amout | Residual amout | Residual amout | Residual amout | Residual amout | Residual amout | Residual amout |
| Total amount | 100m L | 100m L | 100m L | 100m L | 100m L | 100m L | 100m L | 100m L | 100m L | 100m L |
| pH | 5.8 | 6.0 | 5.7 | 6.2 | 5.8 | 5.8 | 5.3 | 5.7 | 6.5 | 5.5 |

[Table 3]

| Component Name | Formulation Example 11 | Formulation Example 12 | Formulation Example 13 | Formulation Example 14 | Formulation Example 15 | Formulation Example 16 |
|---|---|---|---|---|---|---|
| Sodium chondroitin sulfate (Weight-average-molecular weight: 16,000) | 0.2 | 1 | 0.5 | 0.3 | 0.25 | 0.5 |
| Potassium L-aspartate | - | - | 1 | - | 0.5 | 0.5 |
| Magnesium/Potassium L-aspartate | 2 | 1 | - | - | 0.5 | - |
| Taurine | - | 0.5 | - | 1 | - | 0.5 |
| Epsilon-aminocaproic acid | - | - | - | - | 1 | - |
| Potassium chloride | - | - | 0.08 | - | - | 0.05 |
| Sodium chloride | 0.1 | 0.4 | 0.1 | 0.3 | 0.2 | 0.4 |
| Benzalkonium chloride | - | 0.01 | - | - | - | - |
| Chlorhexidine gluconate | - | - | - | - | 0.02 | - |
| Polyhexanide hydrochloride | 1 ppm | - | 1 ppm | - | - | - |
| Chlorobutanol | 0.15 | - | - | - | - | - |
| Zinc chloride | - | - | - | - | - | 0.0001 |
| Sodium edetate | - | 0.005 | 0.05 | 0.02 | 0.01 | 0.05 |
| Boric acid | 1 | 1 | 0.4 | 1 | - | 1 |
| Borax | - | 0.1 | 0.1 | 0.2 | - | 0.2 |
| Sodium hydrogen phosphate | - | - | - | - | 0.15 | - |
| Sodium dihydrogen phosphate | - | - | - | - | 0.1 | - |
| Citric acid | - | - | 0.1 | - | - | 0.1 |
| Concentrated glycerin | - | - | - | - | - | 0.1 |
| Sodium hyaluronate | - | 0.02 | - | - | - | - |
| L-arginine | - | 0.5 | - | - | - | - |
| Polyvinylpyrrolidone | - | - | 0.5 | 0.1 | - | - |
| Hydroxyethyl cellulose | 0.07 | - | - | - | 0.2 | - |
| Hydroxypropyl methylcellulose | - | - | 0.1 | - | 0.2 | 0.3 |
| Hydrochloric acid | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount |

(continued)

| Component Name | Formulation Example 11 | Formulation Example 12 | Formulation Example 13 | Formulation Example 14 | Formulation Example 15 | Formulation Example 16 |
|---|---|---|---|---|---|---|
| Purified water | Residual amount | Residual amount | Residual amount | Residual amount | Residual amount | Residual amount |
| Total amount | 100 mL | 100 mL | 100 mL | 100 mL | 100 mL | 100 mL |
| pH | 6.5 | 7.2 | 6.8 | 7.0 | 7.5 | 6.2 |

**Claims**

1. An ophthalmic composition comprising (A) at least one selected from the group consisting of chondroitin sulfate and a salt thereof, having a weight-average-molecular weight of 1,000 to 20,000, and (B) at least one selected from the group consisting of a water-soluble vitamin, an amino acid, and a salt thereof.

2. The ophthalmic composition according to claim 1, wherein the content of component (A) is 0.001 to 1 w/v% based on the total amount of the ophthalmic composition.

3. The ophthalmic composition according to claim 1, which does not contain a nonionic surfactant.

4. The ophthalmic composition according to claim 1, which is for soft contact lenses.

## INTERNATIONAL SEARCH REPORT

| | International application No. |
| --- | --- |
| | **PCT/JP2024/031846** |

**A. CLASSIFICATION OF SUBJECT MATTER**

*A61K 31/726*(2006.01)i; *A61K 9/08*(2006.01)i; *A61K 47/18*(2017.01)i; *A61K 47/20*(2006.01)i; *A61K 47/22*(2006.01)i; *A61P 27/02*(2006.01)i

FI: A61K31/726; A61P27/02; A61K9/08; A61K47/22; A61K47/18; A61K47/20

According to International Patent Classification (IPC) or to both national classification and IPC

**B. FIELDS SEARCHED**

Minimum documentation searched (classification system followed by classification symbols)

A61K31/726; A61K9/08; A61K47/18; A61K47/20; A61K47/22; A61P27/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2023-112106 A (ROHTO PHARMA) 10 August 2023 (2023-08-10) claims, examples, paragraph [0080] | 1-4 |
| X | JP 2019-199469 A (ROHTO PHARMA) 21 November 2019 (2019-11-21) claims, examples, paragraph [0089] | 1-4 |
| X | JP 2018-83805 A (ROHTO PHARMA) 31 May 2018 (2018-05-31) claims, examples, paragraph [0092] | 1-4 |
| X | JP 2023-52927 A (ROHTO PHARMA) 12 April 2023 (2023-04-12) claims, examples, paragraph [0092] | 1-4 |

☐ Further documents are listed in the continuation of Box C.    ☑ See patent family annex.

| | |
| --- | --- |
| *  Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **22 October 2024** | **05 November 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
**Information on patent family members**

International application No.

**PCT/JP2024/031846**

| Patent document cited in search report | | | Publication date (day/month/year) | Patent family member(s) | Publication date (day/month/year) |
|---|---|---|---|---|---|
| JP | 2023-112106 | A | 10 August 2023 | (Family: none) | |
| JP | 2019-199469 | A | 21 November 2019 | (Family: none) | |
| JP | 2018-83805 | A | 31 May 2018 | (Family: none) | |
| JP | 2023-52927 | A | 12 April 2023 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Over-the-Counter Drug Manufacturing and Sales Approval Standards*, 2017

- Pharmaceutical Excipients Directory. Japan Pharmaceutical Excipients Association, 2016